# EUROPEAN PATENT SPECIFICATION

(11) **EP 1 093 371 B1**
(45) Date of publication and mention of the grant of the patent: **25.05.2005**
(21) Application number: 99926921.0
(22) Date of filing: 02.07.1999
(51) Int. Cl.: A61K 31/445, A61K 31/435, A61P 29/00

(54) **USE OF FK506 AND RELATED MACROLIDES IN THE MANUFACTURE OF A MEDICAMENT FOR TREATMENT OR PREVENTION OF PAIN**
VERWENDUNG VON FK506 UND VERWANDTEN MAKROLIDEN ZUR HERSTELLUNG EINES MEDIKAMENTS FÜR DIE BEHANDLUNG UND VORBEUGUNG VON SCHMERZEN
UTILISATION DE FK506 ET DE MACROLIDES ASSOCIES POUR FABRIQUER UN MEDICAMENT UTILE DANS LE TRAITEMENT OU LA PREVENTION DE LA DOULEUR

(30) Priority: 06.07.1998 GB 9814640
(43) Date of publication of application: 25.04.2001
(73) Proprietor: FUJISAWA PHARMACEUTICAL CO., LTD., Osaka-shi Osaka 541-8514 (JP)
(72) Inventor: KELLY, John, S., Edinburgh EH8 9JZ (GB); MCQUEEN, Daniel, S., Edinburgh EH8 9JZ (GB)
(74) Representative: Gille Hrabal Struck Neidlein Prop Roos
(86) International application number: PCT/JP1999/003602
(87) International publication number: WO 2000/001385

(56) References cited:
- EP-A- 0 664 130
- EP-A- 0 753 297
- WO-A-96/13249
- US-A- 4 859 782

## Description

This invention relates to a new use of FK 506 substance or its hydrate for manufacturing an agent for treating or preventing pain.

### BACKGROUND ART

The macrolide compound and its pharmaceutically acceptable salt for use in accordance with this invention is known to have excellent immunosuppressive activity, and, as such, be of value for the treatment or prevention of rejection reactions by transplantation of organs or tissues, graft-vs.-host diseases, autoimmune diseases, and so on [EP-A-0184162, EP-A-0323042, etc].

US 4,859,782 discloses organic misakinolide compositions which are useful as anti-tumor, anti-viral and anti-fungal compositions.

WO 96/1 3249 discloses a topical composition in the form of an emulsion comprising FK 506 for treating inflammatory or hyperproliferative skin diseases or cutaneous manifestations of immunologically mediated diseases.

EP-A-0 753 297 describes a lotion comprising FK 506 which is useful for treating and preventing inflammatory and proliferative dermatoses and immunologically mediated skin diseases.

EP-A-0 664 130 describes an external preparation for treating hemorrhoidal diseases containing a polyene macrolide fungicide such as pimaricin, nystatin or amphotericin B.

### DISCLOSURE OF INVENTION

The inventors of this invention have surprisingly found that the macrolide compound mentioned herein below has an analgesic effect, especially topical analgesic effect, and thereby is useful as an analgesic.

Accordingly, this invention provides a new use of the macrolide compound for the manufacture of an agent for treating or preventing pain.

The term "macrolide compound" for use in accordance with the invention is the generic name of compounds with 12 members or more, which belong to macrocyclic lactones.

The tricyclic compound mentioned below and its pharmaceutically acceptable salt for use in accordance with this invention are well known to have excellent immunosuppressive activity, antimicrobial activity and other pharmacological activities and, as such, be of value for the treatment or prevention of rejection reactions by transplantation of organs or tissues, graft-vs-host diseases, autoimmune diseases, and infectious diseases [EP-A-0184162, EP-A-0323042, EP-A-423714, EP-A-427680, EP-A-465426, EP-A-480623, EP-A-532088, EP-A-532089, EP-A-569337, EP-A-626385, WO89/05303, WO93/05058, WO96/31514, WO91/13889, WO91/19495, WO93/5059, etc.], the disclosures of which are incorporated herein by reference.

Particularly, the compounds which are designated as FR900506 (=FK506), FR900520 (ascomycin), FR900523, and FR900525 are products produced by microorganisms of the genus Streptomyces, such as Streptomyces tsukubaensis No. 9993 [deposited with National Institute of Bioscience and Human Technology Agency of Industrial Science and Technology (formerly Fermentation Research Institute Agency of Industrial Science and Technology), at 1-3, Higashi 1-chome, Tsukuba-shi, Ibaraki, Japan, date of deposit October 5, 1984, accession number FERM BP-927] or Streptomyces hygroscopicus subsp. yakushimaensis No. 7238 [deposited with National Institute of Bioscience and Human Technology Agency of Industrial Science and Technology (formerly Fermentation Research Institute Agency of Industrial Science and Technology), at 1-3, Higashi 1-chome, Tsukuba-shi, Ibaraki, Japan, date of deposit January 12, 1985, accession number FERM BP-928][EP-A-0184162]. The FK506 (general name: tacrolimus) of the following chemical formula, in particular, is the compound used according to the invention.

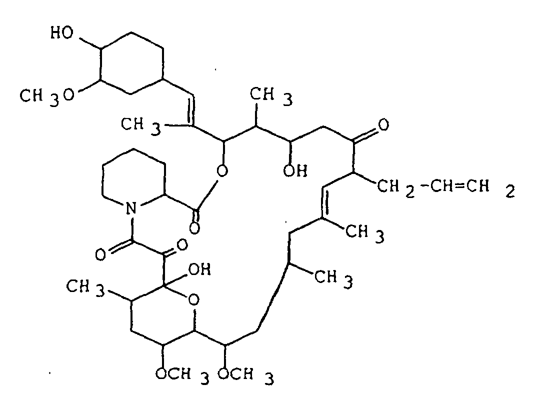

Chemical name: 17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2, 3, 10, 16-tetraone FK 506 substance may be in a form of its salt,
which includes conventional non-toxic and pharmaceutically acceptable salt such as the salt with inorganic or organic bases, specifically, an alkali metal salt such as sodium salt and potassium salt, an alkali earth metal salt such as calcium salt and magnesium salt, an ammonium salt and an amine salt such as triethylamine salt and N-benzyl-N-methylamine salt.

With respect to the macrolide compound used in the present invention, it is to be understood that there may be conformers and one or more stereoisomers such as optical and geometrical isomers due to asymmetric carbon atom(s) or double bond(s), and such conformers and isomers are also included within the scope of macrolide compound in the present invention. And further, the macrolide compound can be in the form of a hydrate , which is included within the scope of the present invention.

The macrolide compound usable in the present invention may be administered as pure compound or mixtures of compounds or preferably, in a pharmaceutical vehicle or carrier.

Pharmaceutical compositions can be used in the form of a pharmaceutical preparation, for example, in solid, semisolid or liquid form, which contains the tricyclic compound used according to the present invention, as an active ingredient, in admixture with an organic or inorganic carrier or excipient suitable for external(topical), enteral, intravenous, intramuscular, or parenteral applications. The active ingredient may be compounded, for example, with the usual non-toxic, pharmaceutically acceptable, carriers for tablets, pellets, capsules, eye drops, suppositories, solutions (saline, for example), emulsion, suspensions (olive oil, for example), ointment, aerosol sprays, cream, skin plasters, patches and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an effective amount sufficient to produce the desired effect upon the process or condition of the disease.

Mammals which may be treated
include livestockmammals such as cows, horses, etc., domestic animals such as dogs, cats, rats, etc. and humans.

For applying this composition to a human, it is preferable to apply it by topical, especially by external, administration, particularly in the form of ointment, gel, lotion, aerosol sprays, cream, skin plasters or patches.

While the dosage of therapeutically effective amount of the macrolide compound varies from and also depends upon the age and condition of each individual patient to be treated, a daily dose of about 0.0001-1000 mg, preferably 0.001-500 mg and more preferably 0.01-100 mg. of the active ingredient is generally given for treating diseases, and an average single dose of about 0.001-0.01mg, 0.2-0.5 mg, 1 mg, 5 mg, 10 mg, 50 mg, 100 mg, 250 mg and 500 mg is generally administered. Daily doses for chronic administration in humans will be in the range of about 0.1-0.3 mg/kg/day.

Especially, when applying externally, the recommended proportion of macrolide compound in the pharmaceutical composition is 0.001~20% (w/w), preferably 0.01~10%(w/w), of the total composition. And further, the macrolide compound can be applied, simultaneously, separately or sequentially, with other agents having analgesic activity or immunosuppressive activity; such as, malononitrilamides (HMR 1279, HMR1715, etc), mycophenolate mofetil (CellCept), steroids, Azathiopurine, and so on.

The following examples illustrate the present invention in further detail.

### Example 1

| | |
|---|---|
| FK506 Substance | 0.1 g |
| propylene carbonate | 5.00 g |
| liquid paraffin | 11.0 g |
| solid paraffin | 3.0 g |
| white bees wax | 3.5 g |
| white petrolatum | q.s. (to 100.0 g) |

The ointment composed of the above ingredients was prepared in a similar manner to that of the Example 1 described in EP-A-0474126.

### Example 2

| | |
|---|---|
| FK 506 Substance | 1 g |
| Hydroxypropyl methylcellulose 2910 (TC-5R) | 1 g |
| Lactose | 2 g |
| Croscarmellose sodium (Ac-Di-Sol) | 1 g |

The FK 506 Substance (1 g) was dissolved in ethanol (10 ml), and thereto was added hydroxypropyl methylcellulose 2910 (TC-5R) (1 g) to prepare a suspension. To this suspension was added dichloromethane (5 ml) to prepare a homogeneous solution. Lactose (2 g) and croscarmellose sodium (Trade Mark: Ac-Di-Sol, maker: Asahi Chemical Industry) were homogeneously suspended to this solution, and then the organic solvent was removed by evaporation. The residual product was dried under reduced pressure for 10 hours by vacuum dryer, milled for 2 minutes by coffee mill and then passed through a sieve (32 mesh) to give the solid dispersion composition of FK 506 Substance (5 g). This composition was capsulated by a conventional manner to provide capsules containing 1 mg or 5 mg of FK 506 Substance per each capsule. This composition can be prepared in a similar manner to that of EP-A-0240773.

### Example 3

### Experiment

Young adult male Lister Hooded rats (Charles Rivers, UK) were maintained under standard animal house conditions, maximum 3 animals per cage, with access to food and water *ad lib*.

Unilateral arthritis was induced using the method described by Donaldson et al, J. Neurosci. Methods 31; 681-691 (1993). Briefly, the rat was anaesthetized with halothane (5% in oxygen) and 0.15 ml of Freund's complete adjuvant (Sigma; 1mg/ml heat killed mycobacterium tuberculosis) injected sub-dermally around the left ankle (tibio-tarsal) joint.

Measurement of pressure evoking reflex withdrawal of the limb when the joint was squeezed was undertaken using an pressure transducer (designed in-house) linked to a chart recorder and the mean of three consecutive pressure measurements made on each ankle, the right (control) joint being measured first. A tape measure was used for determining ankle circumference, and an infrared thermometer held against the joint used to provide a measure of temperature. Rats were weighed to provide an indication of general health.

Drug treatment involved rubbing 75mg (pre-weighed) of ointment A, which is the same one prepared by the above-identified Example 1, or B (vehicle) into the left ankle joint twice daily (at 09:00, 14 :00) for five days. For the acute study, treatment started 24hrs before arthritis was induced (5 rats received FR900506, 5 received vehicle). For the chronic study, treatment started 21 days post-adjuvant, again with 5 rats per group. Measurements were made approximately 10 minutes after the afternoon application of ointment and took 30 minutes to complete.

### Results

The analgesic effect of FR900506 is shown in Fig. 1. FR900506 has analgesic properties when applied topically to chronically hyperalgesic arthritic joints in the rat.

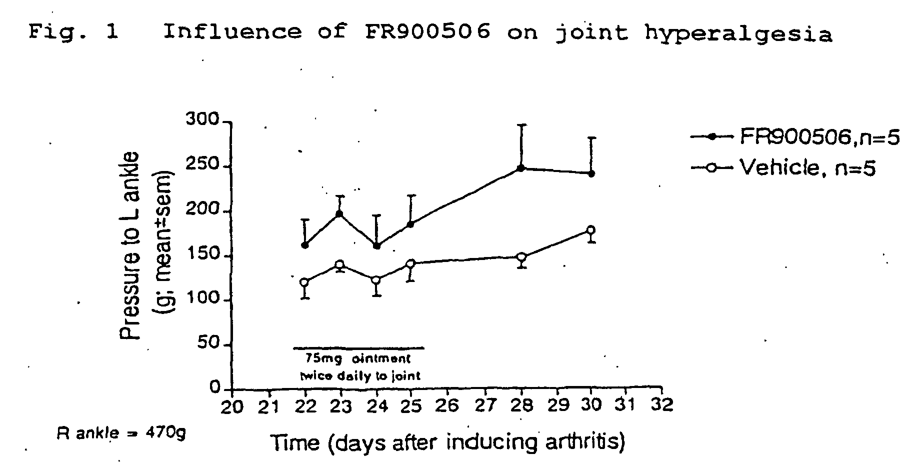

The macrolide compound or its pharmaceutically acceptable salt was proved to have the analgesic effect, especially the topical analgesic effect, and thereby when administered systemically or topically is useful for treating and/or preventing pain(e.g., pain caused by arthritis(rheumatoid arthritis, acute rheumatic arthritis, gouty arthritis, psoriatic arthritis, etc)); arthralgia (intermittent arthralgia, periodic arthralgia, etc); hyperalgesia; allodynia (senile pruritus, etc); cutaneous manifestation of algesthesia caused by various diseases; and so on.

## Claims

1. A use of FK506 substance or its hydrate for manufacturing an agent for treating or preventing pain.

2. The use of claim 1, in which the agent is for treating or preventing pain caused by arthritis.

3. The use of claim 1, in which the agent is for topical administration.

## Patentansprüche

1. Verwendung der FK506-Substanz oder ihres Hydrats zur Herstellung eines Mittels zur Behandlung oder Prävention von Schmerz.

2. Verwendung nach Anspruch 1, wobei das Mittel zur Behandlung oder Prävention von durch Arthritis verursachtem Schmerz vorgesehen ist.

3. Verwendung nach Anspruch 1, wobei das Mittel zur topischen Verabreichung vorgesehen ist.

## Revendications

1. Utilisation de la substance FK506 ou de son hydrate pour la fabrication d'un agent pour traiter ou prévenir une douleur.

2. Utilisation de la revendication 1, dans laquelle l'agent est pour traiter ou prévenir une douleur provoquée par une arthrite.

3. Utilisation de la revendication 1, dans laquelle l'agent est pour une administration topique.
